(19) Europäisches Patentamt
European Patent Office
Office européen des brevets

(11) **EP 2 471 007 B1**

(12) **EUROPEAN PATENT SPECIFICATION**

(45) Date of publication and mention
of the grant of the patent:
**05.02.2020 Bulletin 2020/06**

(51) Int Cl.:
***C12Q 1/686*** [(2018.01)]   ***G06F 17/10*** [(2006.01)]

(21) Application number: **10749782.8**

(86) International application number:
**PCT/EP2010/005196**

(22) Date of filing: **25.08.2010**

(87) International publication number:
**WO 2011/023373 (03.03.2011 Gazette 2011/09)**

(54) **DETERMINATION OF ELBOW VALUES FOR PCR FOR PARABOLIC SHAPED CURVES**

**BESTIMMUNG VON SCHWELLENWERTZYKLEN FÜR PCR-KURVEN MIT PARABOLISCHER FORM**

**DÉTERMINATION DE CYCLES SEUIL POUR COURBES PCR DE FORME PARABOLIQUE**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **26.08.2009 US 548353**

(43) Date of publication of application:
**04.07.2012 Bulletin 2012/27**

(73) Proprietors:
• **Roche Diagnostics GmbH**
  **68305 Mannheim (DE)**
  Designated Contracting States:
  **DE**
• **F. Hoffmann-La Roche AG**
  **4070 Basel (CH)**
  Designated Contracting States:
  **AL AT BE BG CH CY CZ DK EE ES FI FR GB GR HR HU IE IS IT LI LT LU LV MC MK MT NL NO PL PT RO SE SI SK SM TR**

(72) Inventors:
• **KURNIK, Ronald, T.**
  **Foster City, CA 94404 (US)**
• **TITZ, Martin**
  **25704 Meldorf (DE)**

(56) References cited:
**EP-A2- 2 163 999**

• **RITZ R ET AL: "qpcR: an R package for sigmoidal model selection in quantitative real-time polymerase chain reaction analysis", BIOINFORMATICS, vol. 24, no. 13, 14 May 2008 (2008-05-14), pages 1549-1551, XP55042712, ISSN: 1367-4803, DOI: 10.1093/bioinformatics/btn227**
• **RAMAKERS C ET AL: "Assumption-free analysis of quantitative real-time polymerase chain reaction (PCR) data", NEUROSCIENCE LETTERS, vol. 339, no. 1, 13 March 2003 (2003-03-13), pages 62-66, XP002330743, ISSN: 0304-3940, DOI: 10.1016/S0304-3940(02)01423-4**
• **DURTSCHI J D ET AL: "Evaluation of quantification methods for real-time PCR minor groove binding hybridization probe assays", ANALYTICAL BIOCHEMISTRY, vol. 361, no. 1, 5 December 2006 (2006-12-05), pages 55-64, XP005733806, ISSN: 0003-2697, DOI: 10.1016/J.AB.2006.11.023**
• **SPIESS A-N ET AL: "Highly accurate sigmoidal fitting of real-time PCR data by introducing a parameter for asymmetry", BMC BIOINFORMATICS, vol. 9, no. 1, 29 April 2008 (2008-04-29), page 221, XP021031797, ISSN: 1471-2105**
• **SWILLENS S ET AL: "Revisiting the sigmoidal curve fitting applied to quantitative real-time PCR data", ANALYTICAL BIOCHEMISTRY, vol. 373, no. 2, 22 October 2007 (2007-10-22), pages 370-376, XP022411118, ISSN: 0003-2697, DOI: 10.1016/J.AB.2007.10.019**

EP 2 471 007 B1

- RUTLEDGE R G ET AL: "Critical evaluation of methods used to determine amplification efficiency refutes the exponential character of real-time PCR", BMC MOLECULAR BIOLOGY, vol. 9, no. 1, 30 October 2008 (2008-10-30), page 96, XP021042447, ISSN: 1471-2199, DOI: 10.1186/1471-2199-9-96

**Description**

BACKGROUND OF THE INVENTION

The present invention relates generally to systems and methods for processing Polymerase Chain Reaction (PCR) data, and more particularly to systems and methods for determining characteristic cycle threshold (Ct) or elbow values in PCR amplification curves.

The Polymerase Chain Reaction is an in vitro method for enzymatically synthesizing or amplifying defined nucleic acid sequences. The reaction typically uses two oligonucleotide primers that hybridize to opposite strands and flank a template or target DNA sequence that is to be amplified. Elongation of the primers is catalyzed by a heat-stable DNA polymerase. A repetitive series of cycles involving template denaturation, primer annealing, and extension of the annealed primers by the polymerase results in an exponential accumulation of a specific DNA fragment. Fluorescent probes or markers are typically used in the process to facilitate detection and quantification of the amplification process.

In particular, fluorescence techniques that are homogeneous and do not require the addition of reagents after commencement of amplification or physical sampling of the reactions for analysis are attractive. Exemplary homogeneous techniques use oligonucleotide primers to locate the region of interest and fluorescent labels or dyes for signal generation. Typical PCR-based methods use FRET oligonucleotide probes with two interacting chromophores (adjacent hybridization probes, TaqMan probes, Molecular Beacons, Scorpions), single oligonucleotide probes with only one fluorophore (G-quenching probes, Crockett, A. O. and C. T. Wittwer, Anal. Biochem. 2001; 290: 89-97 and SimpleProbes, Idaho Technology), and techniques that use a dsDNA dye (e.g. SYBR Green I) instead of covalent, fluorescently-labeled oligonucleotide probes. DNA-binding dyes that bind to all double-stranded (ds)DNA in PCR cause fluorescence of the dye upon binding. Hence, an increase in DNA product during PCR therefore leads to an increase in fluorescence intensity and is measured at each cycle, thereby allowing DNA concentrations to be quantified. However, a potential drawback is non-specific binding to all dsDNA PCR products impacting precise quantification. With reference to a standard dilution, the dsDNA concentration in the PCR can be determined.

Fluorescent reporter probes detect only the DNA sequence to which the specific probe binds and thus significantly increase specificity. This allows for the quantification of amplificates even in the presence of non-specific DNA amplification. For detection of several targets in the same reaction the fluorescent probes can be used in multiplex assays, wherein specific probes with different-colored labels are used for each target. The specificity of fluorescent reporter probes also prevents interference of measurements caused by primer dimers, which are undesirable by-products during amplification. Most applications are based on the interaction of a fluorescent reporter compound and a quencher of fluorescence that are bound to the probe(s). As long as the reporter and the quencher compound are in close proximity only a basal fluorescence may be detected upon excitation with an excitation source (e.g. a laser, an LED and the like). Once amplification occurs the interaction of the reporter and the quencher compound is disrupted leading to a detectable fluorescent signal. An increase in the product amplified at each PCR cycle causes a proportional increase in fluorescence due to the diminished interaction of the reporter and the quencher compound. Generally, fluorescence is detected and measured in each amplification cycle, and its geometric increase corresponding to exponential increase of the product is used to determine the threshold cycle (CT) in each reaction.

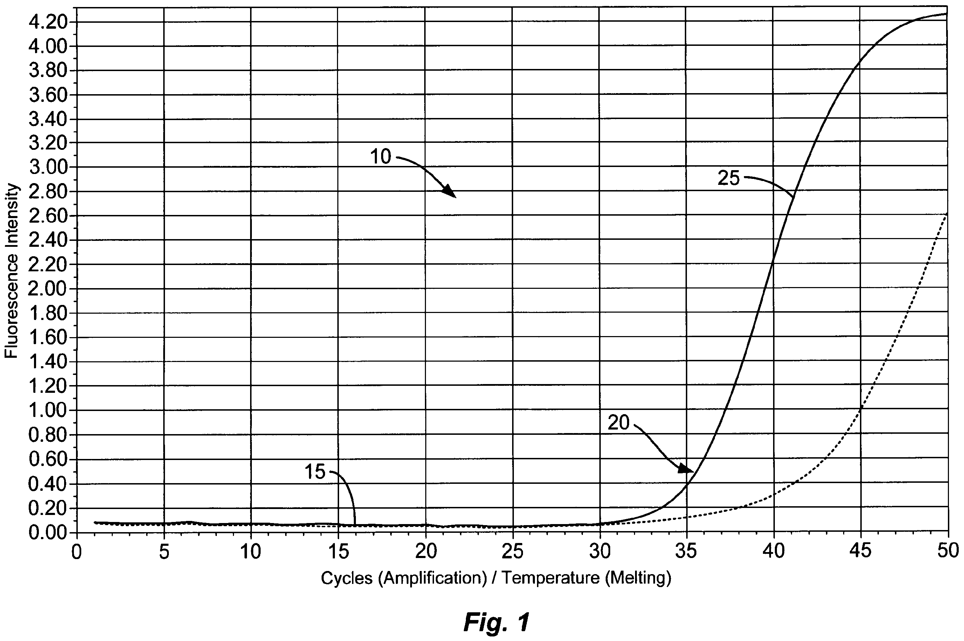

A typical real-time PCR curve is shown in FIG. 1 (solid line), where fluorescence intensity values are plotted vs. cycle number for a typical PCR process. In this case, the formation of PCR products is monitored in each cycle of the PCR process. The amplification is usually measured in thermocyclers which include components and devices for measuring fluorescence signals during the amplification reaction. An example of such a thermocycler is the Roche Diagnostics LightCycler (Cat. No. 20110468). The amplification products are, for example, detected by means of fluorescent labeled hybridization probes which only emit fluorescence signals when they are bound to the target nucleic acid or in certain cases also by means of fluorescent dyes that bind to double-stranded DNA.

For a typical PCR curve, identifying a transition point at the end of the baseline region, which is referred to commonly as the elbow value or cycle threshold (Ct) value, is extremely useful for understanding characteristics of the PCR amplification process. The Ct value may be used as a measure of efficiency of the PCR process. For example, typically a defined signal threshold is determined for all reactions to be analyzed and the number of cycles (Ct) required to reach this threshold value is determined for the target nucleic acid as well as for reference nucleic acids such as a standard or housekeeping gene. The absolute or relative copy numbers of the target molecule can be determined on the basis of the Ct values obtained for the target nucleic acid and the reference nucleic acid (Gibson et al., Genome Research 6:995-1001; Bieche et al., Cancer Research 59:2759-2765, 1999; WO 97/46707; WO 97/46712; WO 97/46714). The elbow value in region 20 at the end of the baseline region 15 in FIG. 1 would be in the region of cycle number 36.

Amounts of RNA or DNA may be determined by comparing the results to a standard curve produced by real-time PCR of serial dilutions of a known amount of RNA or DNA. The absolute or relative copy number of a target molecule in the Polymerase Chain Reaction (PCR) amplification can be determined by comparing the cycle threshold (Ct) value with a standard curve, with the cycle threshold (Ct) value of a reference nucleic acid or with an absolutely quantitated standard nucleic acid. In addition, the efficiency of the Polymerase Chain Reaction (PCR) amplification may be determined by comparing the cycle threshold (Ct) for each of the reactions to be analyzed with the cycle threshold (Ct) of the reference nucleic acid.

The determination of elbows (or cycle thresholds, Ct) for PCR curves is needed for quantitative analysis of real-time PCR (RT-PCR). Many algorithms have been developed for this use, e.g., based on intersection of a normalized data curve with a threshold or by determining the maximum curvature or 2nd derivative, either analytically (e.g., using a curvature algorithm) or numerically, on a normalized data curve, as described in US Application Serial No. 11/316,315, filed December 20, 2005; US Application Serial No. 11/349,550, filed February 6, 2006; US Application Serial No. 11/458644, filed July 19, 2006; US Application Serial No. 11/533,291, filed September 19, 2006; US Application Serial No. 11/861,188, filed September 25, 2007; US Application Serial No 12/209,912, filed September 12, 2008 ("ELCA Algorithm"). These methods work exceedingly well provided that the underlying curve approximates a sigmoid shape. In the rare occasion when the raw data curve has a parabolic shape, then the elbow value as determined by these methods will typically result in an elbow value that is larger than one would expect by examination of the data curve.

Ritz et al., Bioinformatics, vol. 24, no. 13 (May 14, 2008):1549-1551, discloses a software package for determining cycle threshold values for real-time PCR data, wherein a number of different models may be applied to a PCR growth curve in order to select the best model based on measures of goodness of fit, including $R^2$ values and Akaike Information Criterion. However, Ritz et al. does not mention any model assuming that the PCR growth curve may have a parabolic shape. Ramakers et al, Neuroscience Letters, vol. 339, no. 1 (March 13, 2003):62- 66 discloses the use of an iterative algorithm to search for lines consisting of 4 to 6 data points with the highest $R^2$ value and a slope close to the maximum slope to ensure unambiguous selection of data points within the window-of-linearity. However, the window-of-linearity method is not adapted to a parabolic curve shape.

Consider the real-time PCR (RT-PCR) curves from a West Nile Virus (WNV) assay shown in FIG. 1. The solid black curve has a typical sigmoidal shape, which is amendable to analysis with algorithms developed previously. The dashed curve, however, resembles a parabolic curve and lacks a plateau region. The elbow values of the two curves shown in FIG. 1 have the values given in Table 1 below, when analyzed by the ELCA Algorithm as an example. The ELCA Algorithm numerically determines the elbow value as the point of maximum in the curvature or second derivative.

**Table 1: Ct values for Sigmoidal and Parabolic Curves**

|  | ELCA Ct |
| --- | --- |
| Solid Curve | 35.46 |
| Dashed Curve | 45.43 |

The elbow value for the solid curve is consistent with the value one would normally assign to a sigmoid curve, whereas a vertical line drawn at the elbow value for the parabolic dashed curve intersects the dashed curve at a value significantly higher than one would typically assign to this curve.

Accordingly, it is desirable to develop systems and methods to deal with these types of curves, and also to identify when such a parabolic curve is present.

BRIEF SUMMARY OF THE INVENTION

The present invention provides systems and methods to process PCR curves, and to identify the presence of a parabolic-shaped PCR curve. According to the present invention, use of a piecewise linear approximation of a PCR curve enables a more realistic elbow value to be determined in the case of parabolic shaped PCR curves.

According to one aspect of the present invention, a computer-implemented method of determining a cycle threshold (Ct) value for a real-time Polymerase Chain Reaction (PCR) data curve according to independent claim 1 is provided.

According to another aspect of the present invention, a tangible computer readable medium that stores code for controlling a processor to determine a cycle threshold (Ct) value for a real-time Polymerase Chain Reaction (PCR) data curve according to independent claim 7 is provided.

According to yet another aspect of the present invention, a real-time Polymerase Chain Reaction (PCR) according to independent claim 11 is provided.

Embodiments are defined in the dependent claims.

Reference to the remaining portions of the specification, including the drawings and claims, will realize other features and advantages of the present invention. Further features and advantages of the present invention, as well as the structure and operation of various embodiments of the present invention, are described in detail below with respect to the accompanying drawings. In the drawings, like reference numbers indicate identical or functionally similar elements.

BRIEF DESCRIPTION OF THE DRAWINGS

FIG. 1 illustrates a typical growth or amplification curve in the context of a PCR process having a sigmoidal shape (solid line) and a PCR curve having a parabolic shape (dotted line).

FIG. 2 illustrates a parabolic shaped PCR curve and a piecewise linear approximation.

FIG. 3 illustrates a plot of the AIC v. cycle number, from cycle 25 to cycle 49 corresponding to the dotted line (parabolic shaped PCR curve) of Fig. 1.

FIG. 4 illustrates a process for determining a transitionary value in a PCR curve according to one embodiment.

FIG. 5 is an example of general block diagram showing the relation between software and hardware resources that may be used to implement the method and system of the invention

FIG.6 is an example of general block diagram showing the relation between a thermocycler device and a computer system.

DETAILED DESCRIPTION OF THE INVENTION

The present invention provides systems and methods for identifying parabolic-shaped PCR curves and for processing such curves to determine Ct values.
One example of a typical growth or amplification curve 10 in the context of a PCR process having a sigmoidal shape is shown in FIG. 1 as a solid curve. As shown, the curve 10 includes a lag phase region 15, and an exponential phase region 25. Lag phase region 15 is commonly referred to as the baseline or baseline region. Such a curve 10 includes a transitionary region of interest 20 linking the lag phase and the exponential phase regions. Region 20 is commonly referred to as the elbow or elbow region. The elbow region typically defines an end to the baseline and a transition in the growth or amplification rate of the underlying process. Identifying a specific transition point in region 20 can be useful for analyzing the behavior of the underlying process. In a typical PCR curve, identifying a transition point referred to as the elbow value or cycle threshold (Ct) value is useful for understanding qualitative and quantitative characteristics of the PCR process. For example, the Ct value can be used to provide quantization of the amount of DNA present in the sample being analyzed. Quantization is obtained by performing a calibration curve of the Log(DNA Amount) vs. Ct value. Subsequent samples can then use Ct values along with the calibration curve to directly obtain estimates of DNA in a sample. Ct values can also be used to provide qualitative information on the DNA sample.
As shown in FIG. 1, data for a typical PCR growth curve can be represented in a two-dimensional coordinate system, for example, with PCR cycle number defining the x-axis and an indicator of accumulated polynucleotide growth defining the y-axis. Typically, the indicator of accumulated growth is a fluorescence intensity value as the use of fluorescent markers is perhaps the most widely used labeling scheme. However, it should be understood that other indicators may be used depending on the particular labeling and/or detection scheme used. Examples of other useful indicators of accumulated signal growth include luminescence intensity, chemiluminescence intensity, bioluminescence intensity, phosphorescence intensity, charge transfer, voltage, current, power, energy, temperature, viscosity, light scatter, radioactive intensity, reflectivity, transmittance and absorbance. The definition of cycle can also include time, process cycles, unit operation cycles and reproductive cycles.

**General Process Overview**

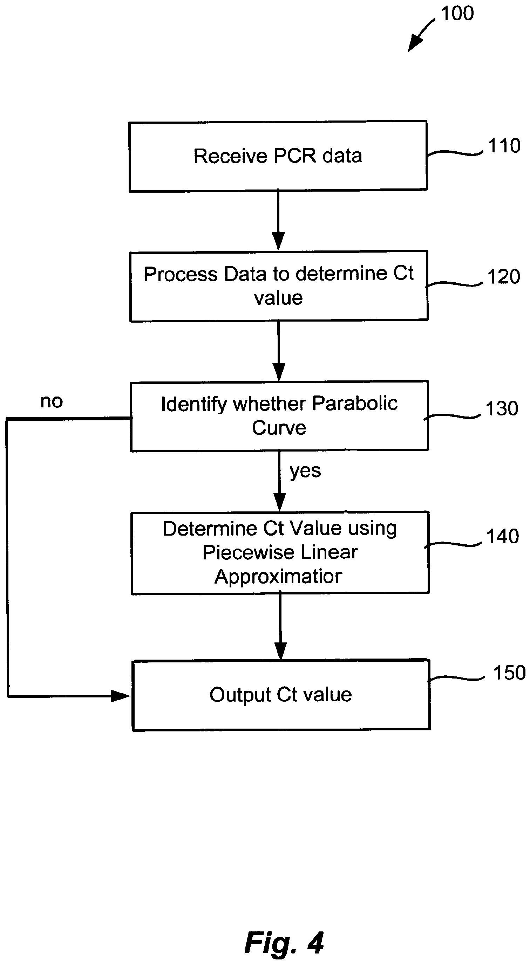

Consider the Real-Time PCR growth curves as shown in FIG. 1. It is desired to obtain from FIG. 1 a number called the Ct or elbow value for each curve. Oftentimes, as shown, real data for a PCR curve results in a parabolic shaped curve for which standard Ct determination processes do not work well. As discussed above with reference to Table 1, the elbow value for the solid curve is consistent with the value one would normally assign to a sigmoid curve, whereas the elbow value for the parabolic dashed curve is significantly higher than one would typically assign to this curve.
According to one embodiment, a process 100 for determining a transitionary value in a PCR curve, namely the elbow value or Ct value of a kinetic PCR amplification curve, can be described briefly with reference to FIG. 4. In step 110, an experimental data set representing the curve is received or otherwise acquired. An example of two plotted PCR data sets is shown in FIG. 1, where the y-axis and x-axis represent fluorescence intensity and cycle number, respectively, for a PCR curve. In certain aspects, the data set should include data that is continuous and equally spaced along an axis.

In the case where process 100 is implemented in an intelligence module (e.g., processor executing instructions) resident in a PCR data acquiring device such as a thermocycler, the data set may be provided to the intelligence module in real time as the data is being collected, or it may be stored in a memory unit or buffer and provided to the intelligence module after the experiment has been completed. Similarly, the data set may be provided to a separate system such as a desktop computer system or other computer system, via a network connection (e.g., LAN, VPN, intranet, Internet, etc.) or direct connection (e.g., USB or other direct wired or wireless connection) to the acquiring device, or provided on a portable medium such as a CD, DVD, floppy disk, memory stick or the like. In certain aspects, the data set includes data points having a pair of coordinate values (or a 2-dimensional vector). For PCR data, the pair of coordinate values typically represents the cycle number and the fluorescence intensity value. After the data set has been received or acquired in step 110, the data set may be analyzed to determine the end of the baseline region.

In step 120, the data set is processed according to a known process to determine a Ct value for the data set/curve. For example, the ELCA process may be used. In step 130, a determination is made as to whether the data set/curve has parabolic shape characteristics. In one embodiment, if all of the following conditions are satisfied, the data set/ curve is determined to have parabolic characteristics and the Ct value is changed to that predicted by equation 4 as will be discussed below:

1. A quadratic fit to the data points beginning from $x^*$ until the end of the given data points is calculated. If the $R^2$ value of this fit is above a given threshold (default = 0.90 or greater), the $C_T$ value is accepted. In one embodiment the threshold is set to 0.99.

2. A $R^2$ value of the piecewise linear fit vs. raw data (over all cycles) > threshold (default = 0.85 or greater). In one embodiment the threshold is set to 0.95.

3. If $a > 0$ and $c > 0$ , the ratio $\dfrac{c}{a}$ is greater than a given threshold (default = 1.1).

If it is determined that the data set/curve does exhibit parabolic characteristics, the process proceeds to step 140.

In step 140, the Ct value for a parabolic curve is determined. To define a more appropriate elbow value to the dashed curve (parabolic curve), a piecewise linear approximation to the RT-PCR curve is used in one embodiment. A piecewise linear function approximation of a parabolic curve according to the present invention is given as follows:

$$f(x) = \begin{cases} ax + b, & x \le x^* \\ c\left(x - x^*\right) + ax^* + b, & x > x^* \end{cases}$$

**Equation 1: Piecewise Linear Equation**

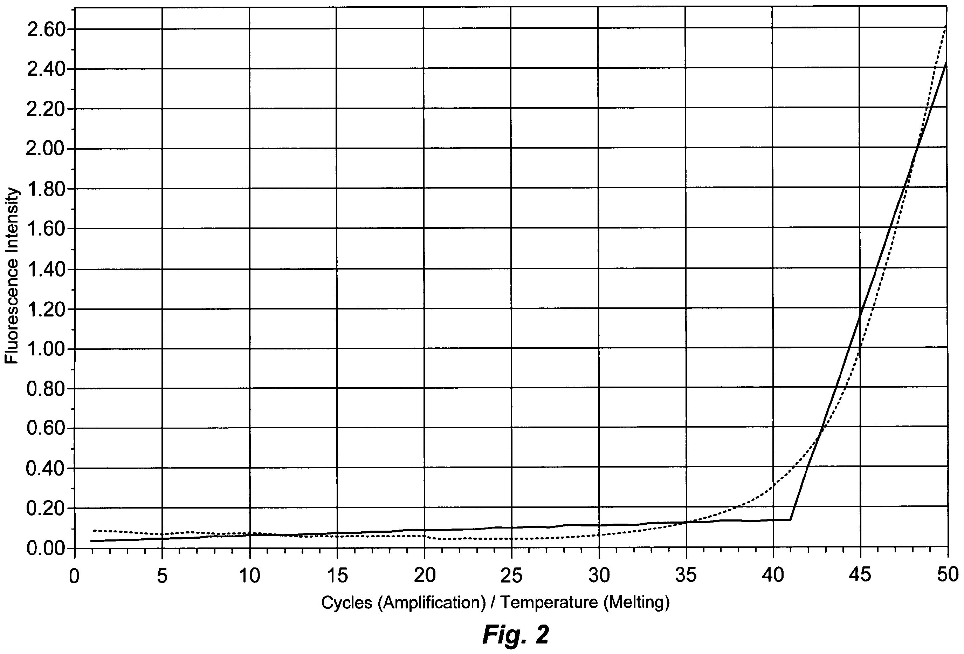

In this equation, the piecewise linear function includes two linear functions joined at a common point, $x^*$. The application of equation 1 is illustrated in FIG. 2, where both the parabolic curve and the piecewise linear approximation are shown.

In one embodiment, the piecewise linear curve is determined by minimization of the objective function shown in Equation 2 at a given value of $x^*$.

$$E(a,b,c) = \frac{1}{2}\sum_{x_i \le x^*}\left(ax_i + b - y_i\right)^2 + \frac{1}{2}\sum_{x_i > x^*}\left(c\left(x_i - x^*\right) + ax^* + b - y_i\right)^2$$

**Equation 2: Objective Function to Minimize**

To determine which value of $x^*$ to use in equation 2, in one embodiment the objective function is applied to all data points from cycles $x^* = n_0...n-1$. In theory $n_0$ can be defined as cycle 1 or cycle 2 or cycle 3, etc., however, to help reduce processing resource consumption, $n_0$ is set at about cycle 20 or 25 or so as these cycle values typically come before the end of the baseline region. The value of $x^*$ which has the minimum Akaike information coefficient (AIC) is chosen. The Akaike information coefficient is defined by:

$$aic = n \cdot \ln\left(\frac{\|\hat{\mathbf{y}} - \mathbf{y}\|^2}{n}\right) + 2 \cdot m + \frac{2 \cdot m \cdot (m+1)}{n-m-1},$$

## Equation 3: Akaike Information Coefficient

where m is the degrees of the freedom of the model, n is the length of the data set, $\mathbf{y}$ is the data vector and $\hat{\mathbf{y}}$ is the predicted model vector. Once the piecewise linear function has been determined, the Ct value is estimated as the intersection of the $2^{nd}$ part of the piecewise linear function with a horizontal line drawn at the mean value of the baseline subtracted raw data curve. Mathematically, this works out as:

$$C_T = x^* + \frac{\underset{i=1\ldots x^*}{mean}(y_i - ax_i - b)}{c - a}$$

## Equation 4: Ct value for Parabolic Curves

In step 150, the Ct value as determined by equation 4 is output or returned, e.g., for display or further processing. Graphical displays may be rendered with a display device, such as a monitor screen or printer, coupled with the system that performed the analysis of FIG. 4, or data may be provided to a separate system for rendering on a display device. However, if, in step 130 it is determined that the data set/curve does not exhibit parabolic characteristics, the Ct value as determined by the process used in step 120 is output in step 150. It should be appreciated that both Ct values could be output if desired. Also, in certain embodiments, for example under the condition that the curve being processed has no plateau, the parabolic Ct determination process of equation 4 could be used as a stand alone algorithm rather than being a subset of another algorithm, such as ELCA, as discussed above (e.g., step 120 of Fig. 4 is omitted).

In a certain embodiment the method according to the invention may be implemented by using conventional personal computer systems including, but not limited to, an input device to input a data set, such as a keyboard, mouse, and the like; a display device to represent a specific point of interest in a region of a curve, such as a monitor; a processing device necessary to carry out each step in the method, such as a CPU; a network interface such as a modem, a data storage device to store the data set, a computer code running on the processor and the like. Furthermore, the method may also be implemented in a PCR analysis module.

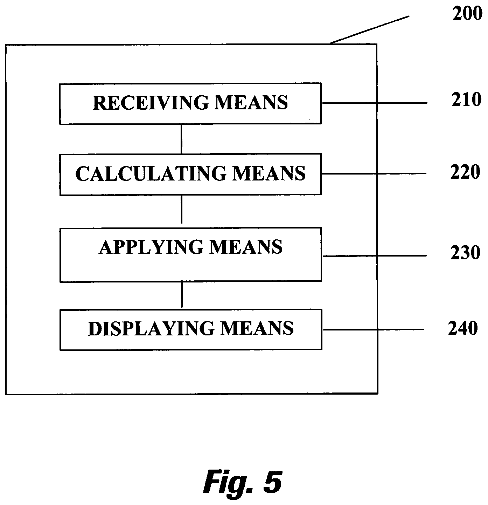

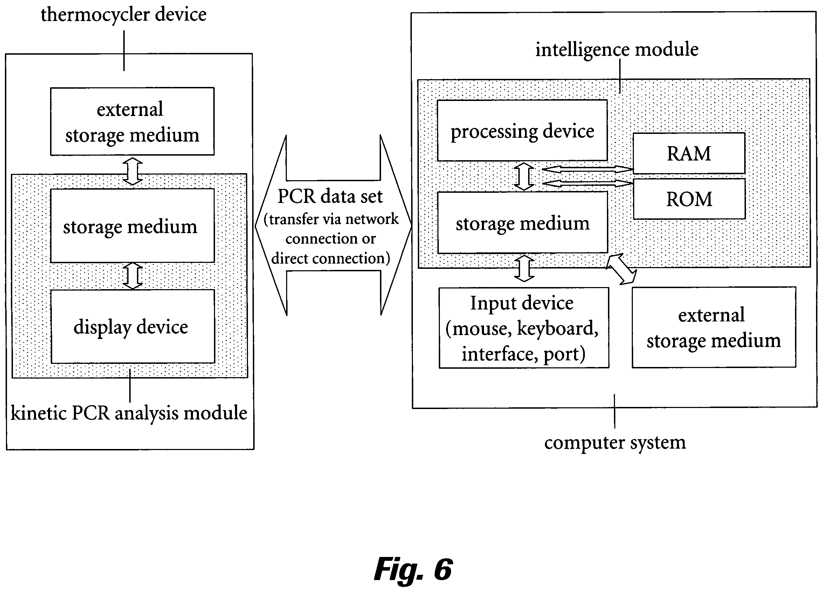

An example of a PCR system is displayed in Fig. 5-6. Fig. 5 shows a general block diagram explaining the relation between software and hardware resources that may be used to implement the method and system of the invention. The system depicted on Fig. 6 comprises a kinetic PCR analysis module which may be located in a thermocycler device and an intelligence module which is part of the computer system. The data sets (PCR data sets) are transferred from the analysis module to the intelligence module or vice versa via a network connection or a direct connection. The data sets may for example be processed according to the flowchart as depicted on Fig. 4. This flowchart may conveniently be implemented by software stored on the hardware of a computer system for example according to the flowchart as depicted on Fig. 5. Referring to Fig. 5, computer system (200) may comprise receiving means (210) for example for receiving fluorescence data obtained during PCR reactions, calculating means (220) for processing said data according to the method of the invention, applying means (230) for replacing a portion of said data according to the results obtained by the calculation means, and displaying means (240) for displaying the results on a computer screen. Fig. 6 illustrates the interaction between the thermocycler device and the computer system. The system comprises a kinetic PCR analysis module which may be located in a thermocycler device and an intelligence module which is part of the computer system.

The data sets (PCR data sets) are transferred from the analysis module to the intelligence module or vice versa via a network connection or a direct connection. The data sets may be processed according to Fig. 5 by computer code running on the processor and being stored on the storage device of the intelligence module and after processing transferred back to the storage device of the analysis module, where the modified data may be displayed on a displaying device.

It should be appreciated that the Ct determination processes, including the parabolic curve determination processes, may be implemented in computer code running on a processor of a computer system or other device or machine (e.g., a thermocycler). The code includes instructions for controlling a processor to implement various aspects and steps of the Ct determination processes. The code is typically stored on a tangible medium such as a hard disk, RAM or portable medium such as a CD, DVD, memory stick, etc. Similarly, the processes may be implemented in a PCR device such as a thermocycler including a processor executing instructions stored in a memory unit integrated in or coupled with the processor. Code including such instructions may be downloaded to the PCR device memory unit over a network connection or direct connection to a code source or using a portable medium as is well known.

One skilled in the art should appreciate that the elbow determination processes of the present invention can be coded using a variety of programming languages such as C, C++, C#, Fortran, VisualBasic, etc., as well as applications such as Mathematica which provide pre-packaged routines, functions and procedures useful for data visualization and analysis. Another example of the latter is MATLAB®.

As explained above, the systems and methods of the invention are useful for determining characteristic cycle threshold (Ct) or elbow values in PCR amplification curves. The determination of elbows (or cycle thresholds, Ct) for PCR curves is needed for quantitative analysis of real-time PCR (RT-PCR). This has a real and tangible utility for example in delivering the outcome of the analysis to patients. In particular, when fluorescence data is used to monitor polymerase chain reactions, the systems and method of the invention provide a more accurate data when the shape of the growth curve is parabolic. Such data is not only useful for monitoring the reaction, but also provides technical effects such as quantification of target nucleic acid amplified during PCR or adapting the reaction conditions of PCR according to the data obtained.

The following examples and figures are provided to aid the understanding of the present invention, the true scope of which is set forth in the appended claims.

EXAMPLE

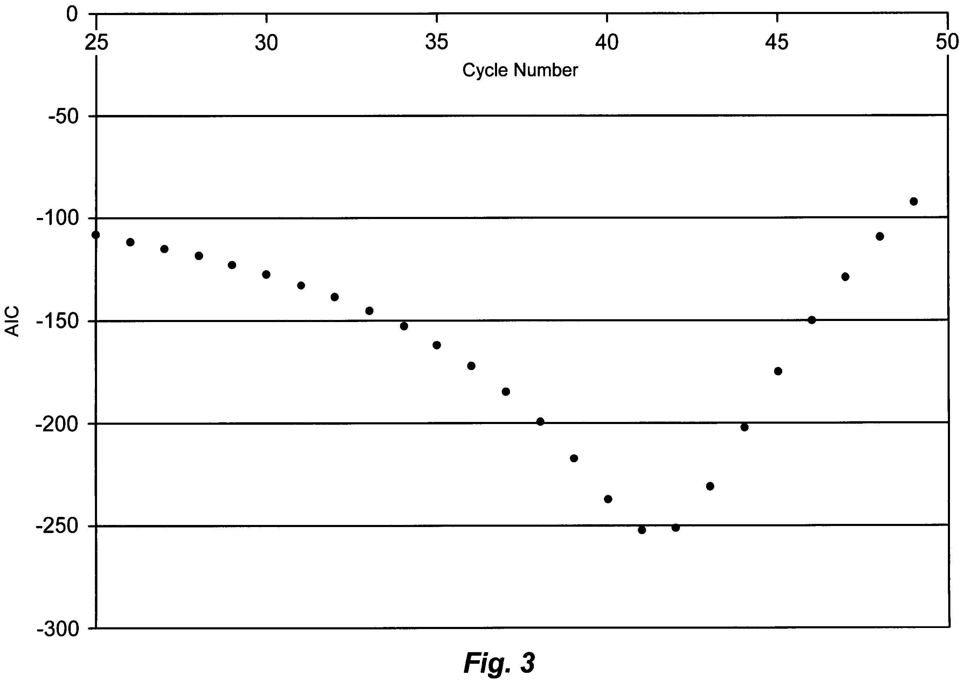

Regression curve fits of Equation 1 were done for x* values from cycles 25 to cycle 49. For each curve fit, the AIC was calculated according to Equation 3. Shown in FIG 3 is a plot of the AIC vs. cycle number, from cycle 25 to cycle 49 corresponding to the dashed curve shown in FIG. 1. The minimum AIC was found to occur at cycle number 41 and the coefficients for Equation 1 using x* = 41 were found to be {a = 0.002472; b = 0.03460; c = 0.25387}. These coefficients were determined by minimizing the function in Equation 2. This piecewise linear fit is accepted as the three conditions were satisfied, namely:

(1) c > 0 and c/a = 102.7, which is greater than the threshold 1.1.

(2) A quadratic fit of the raw data from cycle 41 to cycle 50 has and R2 value of 0.99967, which is greater than the threshold of 0.90 or 0.99.

(3) A piecewise linear fit over all cycle numbers has an R2 value of 0.9872, which is greater then the threshold of 0.85 or 0.95.

Using Equation 4 and the mean baseline subtracted value of the fluorescence = 0.00533, the Ct is then calculated to be Ct = 41.02.

While the invention has been described by way of example and in terms of the specific embodiments, it is to be understood that the invention is not limited to the disclosed embodiments. To the contrary, it is intended to cover various modifications and similar arrangements as would be apparent to those skilled in the art.

**Claims**

1.  A computer-implemented method of determining a cycle threshold (Ct) value for a real-time Polymerase Chain Reaction (PCR) data curve, the method comprising the steps, implemented in a computer system, of:

    - receiving a data set representing a PCR growth curve, the data set having a plurality of coordinate values (x,y), wherein x is a cycle number and y is an indicator of accumulated growth;
    - approximating the data set using a two segment piecewise linear function having a first linear segment ending at x* and a second linear segment beginning at x*, wherein the two segment piecewise linear function has the form:

$$f(x) = \begin{cases} ax + b, & x \le x^* \\ c\left(x - x^*\right) + ax^* + b, & x > x^* \end{cases},$$

    where a, b, and c are parameters of the approximation;

- determining whether the PCR growth curve has a parabolic shape by determining:

a) whether an $R^2$ value of a quadratic fit to the data points beginning with x* to the end of the data set is above a predetermined threshold value of 0.90; and
b) whether an R2 value of the two segment piecewise fit over substantially all the data set is above a threshold value of 0.85; and
c) whether the slope of the second segment is greater than the slope of the first segment if both slopes are greater than zero; and if conditions a), b) and c) are satisfied:

- estimating the Ct value of the data set by applying an equation of the form:

$$C_T = x^* + \frac{\underset{i=1\ldots x^*}{mean}(y_i - ax_i - b)}{c - a},$$

where c is the slope of the second linear segment and b is the y intercept of the first linear segment; and
- outputting the Ct value for display or further processing.

2. The method of claim 1, wherein approximating the data set includes minimizing an objective function at x*, wherein the objective function has the form:

$$E(a,b,c) = \frac{1}{2} \sum_{x_i \leq x^*} (ax_i + b - y_i)^2 + \frac{1}{2} \sum_{x_i > x^*} (c(x_i - x^*) + ax^* + b - y_i)^2.$$

3. The method of claim 2, wherein x* is determined by applying the objective function to all data points from x = n0 to n-1, where n is the length of the data set, n0 is a predetermined starting coordinate, and wherein x*is determined as the value of x which has a minimum Akaike Information Coefficient (AIC).

4. The method of claim 3, wherein the AIC is defined by the equation:

$$aic = n \cdot \ln\left(\frac{\|\hat{\mathbf{y}} - \mathbf{y}\|^2}{n}\right) + 2 \cdot m + \frac{2 \cdot m \cdot (m+1)}{n - m - 1},$$

where m is the number of degrees of the freedom of the model, $\mathbf{y}$ is a data vector and $\hat{\mathbf{y}}$ is a predicted model vector.

5. The method of any one of claims 1 to 4, wherein for determining whether the R2 value of a quadratic fit to the data points beginning with x* to the end of the data set is above the predetermined threshold value, the predetermined threshold value is 0.99.

6. The method of any one of claims 1 to 5, wherein for determining whether the R2 value of the two segment piecewise fit over substantially all the data set is above a threshold value, the threshold value is 0.95.

7. A tangible computer readable medium that stores code for controlling a processor to determine a cycle threshold (Ct) value for a real-time Polymerase Chain Reaction (PCR) data curve, the code including instructions, which when executed by a processor, cause the processor to:

- receive a data set representing a PCR growth curve, the data set having a plurality of coordinate values (x,y), wherein x is a cycle number and y is an indicator of accumulated growth;
- approximate the data set using a two segment piecewise linear function having a first linear segment ending at x* and a second linear segment beginning at x*, wherein the two segment piecewise linear function has the form:

$$f(x) = \begin{cases} ax+b, & x \leq x^* \\ c(x-x^*)+ax^*+b, & x > x^* \end{cases},$$

where a, b, and c are parameters of the approximation;
- determine whether the PCR growth curve has a parabolic shape by determining:

    a) whether an R2 value of a quadratic fit to the data points beginning with x* to the end of the data set is above a threshold value of 0.90; and
    b) whether an R2 value of the two segment piecewise fit over substantially all the data set is above a threshold value of 0.85; and
    c) whether the slope of the second segment is greater than the slope of the first segment if both slopes are greater than zero; and if conditions a), b) and c) are satisfied:

      - estimate the Ct value of the data set by applying an equation of the form:

$$C_T = x^* + \frac{\underset{i=1\dots x^*}{mean}(y_i - ax_i - b)}{c - a},$$

    where c is the slope of the second linear segment and b is the y intercept of the first linear segment; and
    - output the Ct value for display or further processing.

8. The computer readable medium of claim 7, wherein the instructions to approximate the data set include instructions to minimize an objective function at x*, wherein the objective function has the form:

$$E(a,b,c) = \frac{1}{2}\sum_{x_i \leq x^*}(ax_i + b - y_i)^2 + \frac{1}{2}\sum_{x_i > x^*}(c(x_i - x^*)+ax^*+b-y_i)^2.$$

9. The computer readable medium of any one of claims 7 and 8, wherein x* is determined by applying the objective function to all data points from x = n0 to n-1, where n is the length of the data set, n0 is a predetermined starting coordinate, and wherein x* is determined as the value of x which has a minimum Akaike Information Coefficient (AIC).

10. The computer readable medium of claim 9, wherein the AIC is defined by the equation:

$$aic = n \cdot \ln\left(\frac{\|\hat{\mathbf{y}} - \mathbf{y}\|^2}{n}\right) + 2 \cdot m + \frac{2 \cdot m \cdot (m+1)}{n-m-1},$$

where m is the number of degrees of the freedom of the model, $\mathbf{y}$ is a data vector and $\hat{\mathbf{y}}$ is a predicted model vector.

11. A real-time Polymerase Chain Reaction (PCR) system, comprising:

    - a PCR analysis module adapted to generate a PCR data set representing a PCR amplification curve, said data set including a plurality of data points each having a pair of coordinate values (x,y), wherein x is a cycle number and y is an indicator of accumulated growth and wherein said data set includes data points in a region of interest which includes a cycle threshold (Ct) value; and
    - an intelligence module adapted to process the PCR data set to determine the Ct value by:

      - approximating the data set using a two segment piecewise linear function having a first linear segment ending at x* and a second linear segment beginning at x* wherein the two segment piecewise linear function has the form:

$$f(x) = \begin{cases} ax+b, & x \le x^* \\ c\left(x-x^*\right) + ax^* + b, & x > x^* \end{cases},$$

where a, b, and c are parameters of the approximation;
- determining whether the PCR growth curve has a parabolic shape by:

a) determining whether an R2 value of a quadratic fit to the data points beginning with x* to the end of the data set is above a threshold value of 0.90; and
b) determining whether an R2 value of the two segment piecewise fit over substantially all the data set is above a threshold value of 0.85; and
c) determining whether the slope of the second segment is greater than the slope of the first segment if both slopes are greater than zero; and if conditions a), b) and c) are satisfied :
- - estimating the Ct value of the data set by applying an equation of the form:

$$C_T = x^* + \frac{\underset{i=1\ldots x^*}{mean}\left(y_i - ax_i - b\right)}{c - a},$$

where c is the slope of the second linear segment and b is the y intercept of the first linear segment; and

- outputting the Ct value for display or further processing.

12. The PCR system of claim 11, wherein approximating the data set includes minimizing an objective function at x*, wherein the objective function has the form:

$$E(a,b,c) = \frac{1}{2}\sum_{x_i \le x^*}\left(ax_i + b - y_i\right)^2 + \frac{1}{2}\sum_{x_i > x^*}\left(c\left(x_i - x^*\right) + ax^* + b - y_i\right)^2.$$

13. The PCR system of any one of claims 11 and 12, wherein x* is determined by applying the objective function to all data points from x = n0 to n-1, where n is the length of the data set, n0 is a predetermined starting coordinate, and wherein x*is determined as the value of x which has a minimum Akaike Information Coefficient (AIC).

14. The PCR system of claim 13, wherein the AIC is defined by the equation:

$$aic = n \cdot \ln\left(\frac{\|\hat{\mathbf{y}} - \mathbf{y}\|^2}{n}\right) + 2 \cdot m + \frac{2 \cdot m \cdot (m+1)}{n - m - 1},$$

where m is the number of degrees of the freedom of the model, $\mathbf{y}$ is a data vector and $\hat{\mathbf{y}}$ is a predicted model vector.

**Patentansprüche**

1. Computerimplementiertes Verfahren zum Bestimmen eines Zyklusschwellenwertes (Ct) für eine Echtzeit-Datenkurve einer Polymerasekettenreaktion (PCR), wobei das Verfahren, implementiert in einem Computersystem, die folgenden Schritte umfasst:

- Empfangen eines Datensatzes, der eine PCR-Wachstumskurve darstellt, wobei der Datensatz eine Vielzahl von Koordinatenwerten (x,y) aufweist, wobei x eine Zyklenzahl ist und y ein Indikator für akkumuliertes Wachstum ist;
- Näherung des Datensatzes unter Anwendung einer stückweisen linearen Funktion mit zwei Segmenten, die ein erstes lineares Segment, das bei x* endet, und ein zweites lineares Segment, das bei x* beginnt, aufweist, wobei die stückweise lineare Funktion mit zwei Segmenten folgendermaßen lautet:

$$f(x) = \begin{cases} ax + b, & x \leq x^* \\ c(x - x^*) + ax^* + b, & x > x^* \end{cases},$$

wobei a, b und c Parameter der Näherung sind;
- Bestimmen, ob die PCR-Wachstumskurve eine parabolische Form hat, indem bestimmt wird:

a) ob ein $R^2$-Wert einer quadratischen Anpassung an die Datenpunkte, beginnend mit x* bis zum Ende des Datensatzes, über einem vorbestimmten Schwellenwert von 0,90 liegt; und
b) ob ein R2-Wert der stückweisen Anpassung mit zwei Segmenten über im Wesentlichen den gesamten Datensatz über einem Schwellenwert von 0,85 liegt; und
c) ob die Steigung des zweiten Segments größer ist als die Steigung des ersten Segments, wenn beide Steigungen größer als null sind; und, wenn die Bedingungen a), b) und c) erfüllt sind:

- Schätzen des Ct-Wertes des Datensatzes durch Anwenden einer Gleichung der folgenden Form:

$$C_T = x^* + \frac{\underset{i=1...x^*}{Mittel}(y_i - ax_i - b)}{c - a},$$

wobei c die Steigung des zweiten linearen Segments ist und b der y-Achsenabschnitt des ersten linearen Segments ist; und
- Ausgeben des Ct-Wertes zur Anzeige oder Weiterverarbeitung.

2. Verfahren nach Anspruch 1, wobei die Näherung des Datensatzes das Minimieren einer Zielfunktion bei x* einschließt, wobei die Zielfunktion folgendermaßen lautet:

$$E(a,b,c) = \frac{1}{2} \sum_{x_i \leq x^*} (ax_i + b - y_i)^2 + \frac{1}{2} \sum_{x_i > x^*} (c(x_i - x^*) + ax^* + b - y_i)^2.$$

3. Verfahren nach Anspruch 2, wobei x* durch Anwenden der Zielfunktion auf alle Datenpunkte von x = n0 bis n-1 umfasst, wobei n die Länge des Datensatzes ist, n0 eine vorbestimmte Ausgangskoordinate ist, und wobei x* als der Wert von x bestimmt wird, der über ein minimales Akaike-Informationskriterium (AIC) verfügt.

4. Verfahren nach Anspruch 3, wobei das AIC durch die folgende Gleichung definiert ist:

$$aic = n \cdot \ln\left(\frac{\|\hat{\mathbf{y}} - \mathbf{y}\|^2}{n}\right) + 2 \cdot m + \frac{2 \cdot m \cdot (m+1)}{n - m - 1},$$

wobei m die Anzahl von Freiheitsgraden des Modells ist, y ein Datenvektor ist und $\hat{y}$ ein vorhergesagter Modellvektor ist.

5. Verfahren nach einem der Ansprüche 1 bis 4, wobei zum Bestimmen, ob der R2-Wert einer quadratischen Anpassung an die Datenpunkte, beginnend mit x* bis zum Ende des Datensatzes, über dem vorbestimmten Schwellenwert liegt, der vorbestimmte Schwellenwert 0,99 beträgt.

6. Verfahren nach einem der Ansprüche 1 bis 5, wobei zum Bestimmen, ob der R2-Wert der stückweisen Anpassung mit zwei Segmenten über im Wesentlichen den gesamten Datensatz über einem Schwellenwert liegt, der Schwellenwert 0,95 beträgt.

**7.** Greifbares, computerlesbares Medium, das einen Code zum Steuern eines Prozessors zum Bestimmen eines Zyklusschwellenwertes (Ct) für eine Echtzeit-Datenkurve einer Polymerasekettenreaktion (PCR) speichert, wobei der Code Anweisungen einschließt, die, ausgeführt von einem Prozessor, den Prozessor veranlassen:

- einen Datensatz zu empfangen, der eine PCR-Wachstumskurve darstellt, wobei der Datensatz eine Vielzahl von Koordinatenwerten (x,y) aufweist, wobei x eine Zyklenzahl ist und y ein Indikator für akkumuliertes Wachstum ist;
- den Datensatz unter Anwendung einer stückweisen linearen Funktion mit zwei Segmenten, die ein erstes lineares Segment, das bei x* endet, und ein zweites lineares Segment, das bei x* beginnt, aufweist, zu nähern, wobei die stückweise lineare Funktion mit zwei Segmenten folgendermaßen lautet:

$$f(x) = \begin{cases} ax + b, & x \le x^* \\ c\left(x - x^*\right) + ax^* + b, & x > x^* \end{cases},$$

wobei a, b und c Parameter der Näherung sind;
- zu bestimmen, ob die PCR-Wachstumskurve eine parabolische Form hat, indem bestimmt wird:

a) ob ein R2-Wert einer quadratischen Anpassung an die Datenpunkte, beginnend mit x* bis zum Ende des Datensatzes, über einem vorbestimmten Schwellenwert von 0,90 liegt; und
b) ob ein R2-Wert der stückweisen Anpassung mit zwei Segmenten über im Wesentlichen den gesamten Datensatz über einem Schwellenwert von 0,85 liegt; und
c) ob die Steigung des zweiten Segments größer ist als die Steigung des ersten Segments, wenn beide Steigungen größer als null sind; und, wenn die Bedingungen a), b) und c) erfüllt sind:

- den Ct-Wert des Datensatzes durch Anwenden einer Gleichung der folgenden Form zu schätzen:

$$C_T = x^* + \frac{\underset{i=1\ldots x^*}{Mittel}\left(y_i - ax_i - b\right)}{c - a},$$

wobei c die Steigung des zweiten linearen Segments ist und b der y-Achsenabschnitt des ersten linearen Segments ist; und
- den Ct-Wert zum Anzeigen oder zur Weiterverarbeitung auszugeben.

**8.** Computerlesbares Medium nach Anspruch 7, wobei die Anweisungen zum Nähern des Datensatzes Anweisungen zum Minimieren einer Zielfunktion bei x* einschließen, wobei die Zielfunktion folgendermaßen lautet:

$$E(a,b,c) = \frac{1}{2}\sum_{x_i \le x^*}\left(ax_i + b - y_i\right)^2 + \frac{1}{2}\sum_{x_i > x^*}\left(c\left(x_i - x^*\right) + ax^* + b - y_i\right)^2.$$

**9.** Computerlesbares Medium nach einem der Ansprüche 7 und 8, wobei x* durch Anwenden der Zielfunktion auf alle Datenpunkte von x = n0 bis n-1 bestimmt wird, wobei n die Länge des Datensatzes ist, n0 eine vorbestimmte Ausgangskoordinate ist, und wobei x* als der Wert von x bestimmt wird, der über ein minimales Akaike-Informationskriterium (AIC) verfügt.

**10.** Computerlesbares Medium nach Anspruch 9, wobei das AIC durch die folgende Gleichung definiert ist:

$$aic = n \cdot \ln\left(\frac{\|\hat{\mathbf{y}} - \mathbf{y}\|^2}{n}\right) + 2 \cdot m + \frac{2 \cdot m \cdot (m+1)}{n - m - 1},$$

wobei m die Anzahl von Freiheitsgraden des Modells ist, **y** ein Datenvektor ist und **ŷ** ein vorhergesagter Modellvektor

ist.

11. Echtzeit-Polymerasekettenreaktion-(PCR)-System, umfassend:

- ein PCR-Analysemodul, das ausgelegt ist, einen PCR-Datensatz zu erzeugen, der eine PCR-Amplifikationskurve darstellt, wobei der Datensatz eine Vielzahl von Datenpunkten einschließt, die jeweils zwei Koordinatenwerte (x,y) aufweisen, wobei x eine Zyklenzahl ist und y ein Indikator für akkumuliertes Wachstum ist, und wobei der Datensatz Datenpunkte in einer relevanten Region einschließt, der einen Zyklusschwellenwert (Ct) einschließt; und
- ein Intelligenzmodul, das ausgelegt ist, den PCR-Datensatz zu verarbeiten, um den Ct-Wert zu bestimmen, indem:

- der Datensatz unter Anwendung einer stückweisen linearen Funktion mit zwei Segmenten, die ein erstes lineares Segment, das bei x* endet, und ein zweites lineares Segment, das bei x* beginnt, aufweist, genähert wird, wobei die stückweise lineare Funktion mit zwei Segmenten folgendermaßen lautet:

$$f(x) = \begin{cases} ax+b, & x \le x^* \\ c\left(x-x^*\right)+ax^*+b, & x > x^* \end{cases},$$

wobei a, b und c Parameter der Näherung sind;
- bestimmt wird, ob die PCR-Wachstumskurve eine parabolische Form hat, indem:

a) bestimmt wird, ob ein R2-Wert einer quadratischen Anpassung an die Datenpunkte, beginnend mit x* bis zum Ende des Datensatzes, über einem Schwellenwert von 0,90 liegt; und
b) bestimmt wird, ob ein R2-Wert der stückweisen Anpassung mit zwei Segmenten über den gesamten Datensatz über einem Schwellenwert von 0,85 liegt; und
c) bestimmt wird, ob die Steigung des zweiten Segments größer ist als die Steigung des ersten Segments, wenn beide Steigungen größer sind als null; und, wenn die Bedingungen a), b) und c) erfüllt sind:

- der Ct-Wert des Datensatzes geschätzt wird, indem folgende Gleichung angewandt wird:

$$C_T = x^* + \frac{Mittel_{i=1,..x^*}\left(y_i - ax_i - b\right)}{c-a},$$

wobei c die Steigung des zweiten linearen Segments ist und b der y-Achsenabschnitt des ersten linearen Segments ist; und
- der Ct-Wert zum Anzeigen oder zur Weiterverarbeitung ausgegeben wird.

12. PCR-System nach Anspruch 11, wobei die Näherung des Datensatzes das Minimieren einer Zielfunktion bei x* einschließt, wobei die Zielfunktion folgendermaßen lautet:

$$E\left(a,b,c\right) = \frac{1}{2}\sum_{x_i \le x^*}\left(ax_i+b-y_i\right)^2 + \frac{1}{2}\sum_{x_i > x^*}\left(c\left(x_i - x^*\right)+ax^*+b-y_i\right)^2.$$

13. PCR-System nach einem der Ansprüche 11 und 12, wobei x* durch Anwenden der Zielfunktion auf alle Datenpunkte von x = n0 bis n-1 bestimmt wird, wobei n die Länge des Datensatzes ist, n0 eine vorbestimmte Ausgangskoordinate ist, und wobei x* als der Wert von x bestimmt wird, der über ein minimales Akaike-Informationskriterium (AIC) verfügt.

14. PCR-System nach Anspruch 13, wobei das AIC durch die folgende Gleichung definiert ist:

$$aic = n \cdot \ln\left(\frac{\|\hat{\mathbf{y}} - \mathbf{y}\|^2}{n}\right) + 2 \cdot m + \frac{2 \cdot m \cdot (m+1)}{n-m-1},$$

wobei m die Anzahl von Freiheitsgraden des Modells ist, $\mathbf{y}$ ein Datenvektor ist und $\hat{\mathbf{y}}$ ein vorhergesagter Modellvektor ist.

**Revendications**

1. Procédé mis en œuvre par ordinateur de détermination d'une valeur de cycle seuil (Ct) pour une courbe de données de réaction en chaîne par polymérase (PCR) en temps réel, le procédé comprenant les étapes, mises en œuvre sur un système informatique, de :

   - réception d'un jeu de données représentant une courbe de croissance par PCR, le jeu de données ayant une pluralité de valeurs de coordonnées (x,y), dans lequel x est un nombre de cycles et y est un indicateur de la croissance accumulée ;
   - approximation du jeu de données en utilisant une fonction linéaire par morceaux de deux segments ayant un premier segment linéaire se terminant au niveau de x* et un second segment linéaire commençant au niveau de x*, dans lequel la fonction linéaire par morceaux de deux segments a la forme :

$$f(x) = \begin{cases} ax + b, & x \leq x^* \\ c(x - x^*) + ax^* + b, & x > x^* \end{cases},$$

   où a, b et c sont des paramètres de l'approximation ;
   - détermination si la courbe de croissance par PCR a une forme parabolique en déterminant :

   a) si une valeur $R^2$ d'un ajustement quadratique aux points de données commençant avec x* jusqu'à la fin du jeu de données est supérieure à une valeur seuil prédéterminée de 0,90 ; et
   b) si une valeur R2 de l'ajustement par morceaux de deux segments sur substantiellement la totalité du jeu de données est supérieure à une valeur seuil de 0,85 ; et
   c) si la pente du second segment est supérieure à la pente du premier segment lorsque les deux pentes sont supérieures à zéro ; et si les conditions a), b) et c) sont remplies :

   - estimation de la valeur Ct du jeu de données par application d'une équation de la forme :

$$C_T = x^* + \frac{\underset{i=1\ldots x^*}{moyenne}(y_i - ax_i - b)}{c - a},$$

   où c est la pente du second segment linéaire et b est l'ordonnée à l'origine y du premier segment linéaire ; et
   - production de la valeur Ct pour affichage ou traitement supplémentaire.

2. Procédé selon la revendication 1, dans lequel l'approximation du jeu de données inclut la minimisation d'une fonction objective au niveau de x*, dans lequel la fonction objective a la forme :

$$E(a,b,c) = \frac{1}{2}\sum_{x_i \leq x^*}(ax_i + b - y_i)^2 + \frac{1}{2}\sum_{x_i > x^*}(c(x_i - x^*) + ax^* + b - y_i)^2 .$$

**3.** Procédé selon la revendication 2, dans lequel x* est déterminé par application de la fonction objective à tous les points de données de x = n0 à n-1, où n est la longueur du jeu de données, n0 est une coordonnée de départ prédéterminée, et dans lequel x* est déterminé comme étant la valeur de x qui présente un coefficient d'information d'Akaike (AIC) minimal.

**4.** Procédé selon la revendication 3, dans lequel l'AIC est défini par l'équation :

$$aic = n \cdot \ln\left(\frac{\|\hat{\mathbf{y}} - \mathbf{y}\|^2}{n}\right) + 2 \cdot m + \frac{2 \cdot m \cdot (m+1)}{n - m - 1},$$

où m est le nombre de degrés de liberté du modèle, **y** est un vecteur de données et $\hat{\mathbf{y}}$ est un vecteur modèle prédit.

**5.** Procédé selon l'une quelconque des revendications 1 à 4, dans lequel pour déterminer si la valeur R2 d'un ajustement quadratique aux points de données commençant par x* jusqu'à la fin du jeu de données est supérieure à la valeur seuil prédéterminée, la valeur seuil prédéterminée est 0,99.

**6.** Procédé selon l'une quelconque des revendications 1 à 5, dans lequel pour déterminer si la valeur R2 de l'ajustement par morceaux de deux segments sur substantiellement la totalité du jeu de données est supérieure à une valeur seuil, la valeur seuil est 0,95.

**7.** Support lisible par ordinateur tangible qui stocke un code pour commander un processeur afin de déterminer une valeur de cycle seuil (Ct) pour une courbe de données de réaction en chaîne par polymérase (PCR) en temps réel, le code incluant des instructions, qui lorsqu'elles sont exécutées par un processeur, entraînent le processeur à :

- recevoir un jeu de données représentant une courbe de croissance par PCR, le jeu de données ayant une pluralité de valeurs de coordonnées (x,y), dans lequel x est un nombre de cycles et y est un indicateur de la croissance accumulée ;
- approximer le jeu de données en utilisant une fonction linéaire par morceaux de deux segments ayant un premier segment linéaire se terminant au niveau de x* et un second segment linéaire commençant au niveau de x*, dans lequel la fonction linéaire par morceaux de deux segments a la forme :

$$f(x) = \begin{cases} ax + b, & x \leq x^* \\ c(x - x^*) + ax^* + b, & x > x^* \end{cases},$$

où a, b et c sont des paramètres de l'approximation ;
- déterminer si la courbe de croissance par PCR a une forme parabolique en déterminant :

a) si une valeur R2 d'un ajustement quadratique aux points de données commençant avec x* jusqu'à la fin du jeu de données est supérieure à une valeur seuil de 0,90 ; et
b) si une valeur R2 de l'ajustement par morceaux de deux segments sur substantiellement la totalité du jeu de données est supérieure à une valeur seuil de 0,85 ; et
c) si la pente du second segment est supérieure à la pente du premier segment lorsque les deux pentes sont supérieures à zéro ; et si les conditions a), b) et c) sont remplies :

- estimer la valeur Ct du jeu de données par application d'une équation de la forme :

$$C_T = x^* + \frac{\underset{i=1\ldots x^*}{moyenne}\left(y_i - ax_i - b\right)}{c-a}\,,$$

où c est la pente du second segment linéaire et b est l'ordonnée à l'origine y du premier segment linéaire ; et
- produire la valeur Ct pour affichage ou traitement supplémentaire.

8. Support lisible par ordinateur selon la revendication 7, dans lequel les instructions d'approximation du jeu de données incluent des instructions de minimisation d'une fonction objective au niveau de x*, dans lequel la fonction objective a la forme :

$$E(a,b,c) = \frac{1}{2}\sum_{x_i \le x^*}\left(ax_i + b - y_i\right)^2 + \frac{1}{2}\sum_{x_i > x^*}\left(c\left(x_i - x^*\right) + ax^* + b - y_i\right)^2.$$

9. Support lisible par ordinateur selon l'une quelconque des revendications 7 et 8, dans lequel x* est déterminé par application de la fonction objective à tous les points de données de x = n0 à n-1, où n est la longueur du jeu de données, n0 est une coordonnée de départ prédéterminée, et dans lequel x* est déterminé comme étant la valeur de x qui présente un coefficient d'information d'Akaike (AIC) minimal.

10. Support lisible par ordinateur selon la revendication 9, dans lequel l'AIC est défini par l'équation :

$$aic = n \cdot \ln\left(\frac{\|\hat{\mathbf{y}} - \mathbf{y}\|^2}{n}\right) + 2 \cdot m + \frac{2 \cdot m \cdot (m+1)}{n-m-1}\,,$$

où m est le nombre de degrés de liberté du modèle, **y** est un vecteur de données et $\hat{\mathbf{y}}$ est un vecteur modèle prédit.

11. Système de réaction en chaîne par polymérase (PCR) en temps réel, comprenant :

- un module d'analyse par PCR adapté à la génération d'un jeu de données de PCR représentant une courbe d'amplification par PCR, ledit jeu de données incluant une pluralité de points de données chacun ayant une paire de valeurs de coordonnées (x,y), dans lequel x est un nombre de cycles et y est un indicateur de la croissance accumulée et dans lequel ledit jeu de données inclut des points de données dans une région d'intérêt qui inclut une valeur de cycle seuil (Ct) ; et
- un module d'intelligence adapté au traitement du jeu de données de PCR pour déterminer la valeur Ct par :

- approximation du jeu de données en utilisant une fonction linéaire par morceaux de deux segments ayant un premier segment linéaire se terminant au niveau de x* et un second segment linéaire commençant au niveau de x* dans lequel la fonction linéaire par morceaux de deux segments est de la forme :

$$f(x) = \begin{cases} ax+b, & x \le x^* \\ c\left(x-x^*\right) + ax^* + b, & x > x^* \end{cases},$$

où a, b et c sont des paramètres de l'approximation ;
- détermination si la courbe de croissance par PCR a une forme parabolique en :

a) déterminant si une valeur R2 d'un ajustement quadratique aux points de données commençant avec x* jusqu'à la fin du jeu de données est supérieure à une valeur seuil de 0,90 ; et
b) déterminant si une valeur R2 de l'ajustement par morceaux de deux segments sur substantiellement la totalité du jeu de données est supérieure à une valeur seuil de 0,85 ; et
c) déterminant si la pente du second segment est supérieure à la pente du premier segment lorsque les deux pentes sont supérieures à zéro ; et si les conditions a), b) et c) sont remplies :

- estimation de la valeur Ct du jeu de données par application d'une équation de la forme :

$$C_T = x^* + \frac{\underset{i=1...x^*}{moyenne}\left(y_i - ax_i - b\right)}{c - a},$$

où c est la pente du second segment linéaire et b est l'ordonnée à l'origine y du premier segment linéaire ; et
- production de la valeur Ct pour affichage ou traitement supplémentaire.

**12.** Système de PCR selon la revendication 11, dans lequel l'approximation du jeu de données inclut la minimisation d'une fonction objective au niveau de x*, dans lequel la fonction objective a la forme :

$$E(a,b,c) = \frac{1}{2}\sum_{x_i \leq x^*}\left(ax_i + b - y_i\right)^2 + \frac{1}{2}\sum_{x_i > x^*}\left(c(x_i - x^*) + ax^* + b - y_i\right)^2.$$

**13.** Système de PCR selon l'une quelconque des revendications 11 et 12, dans lequel x* est déterminé par application de la fonction objective à tous les points de données de x = n0 à n-1, où n est la longueur du jeu de données, n0 est une coordonnée de départ prédéterminée, et dans lequel x* est déterminé comme étant la valeur de x qui présente un coefficient d'information d'Akaike (AIC) minimal.

**14.** Système de PCR selon la revendication 13, dans lequel l'AIC est défini par l'équation :

$$aic = n \cdot \ln\left(\frac{\|\hat{\mathbf{y}} - \mathbf{y}\|^2}{n}\right) + 2 \cdot m + \frac{2 \cdot m \cdot (m+1)}{n - m - 1},$$

où m est le nombre de degrés de liberté du modèle, **y** est un vecteur de données et $\hat{\mathbf{y}}$ est un vecteur modèle prédit.

**Fig. 1**

EP 2 471 007 B1

*Fig. 2*

*Fig. 3*

EP 2 471 007 B1

_100_

Receive PCR data — 110

Process Data to determine Ct value — 120

no → Identify whether Parabolic Curve — 130

yes

Determine Ct Value using Piecewise Linear Approximatior — 140

Output Ct value — 150

*Fig. 4*

Fig. 5

*Fig. 6*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9746707 A
- WO 9746712 A
- WO 9746714 A
- US 31631505
- US 34955006
- US 45864406
- US 53329106
- US 86118807
- US 20991208

**Non-patent literature cited in the description**

- **G-QUENCHING PROBES ; CROCKETT, A. O. ; C. T. WITTWER.** *Anal. Biochem.,* 2001, vol. 290, 89-97
- **GIBSON et al.** *Genome Research,* vol. 6, 995-1001
- **BIECHE et al.** *Cancer Research,* 1999, vol. 59, 2759-2765
- **RITZ et al.** *Bioinformatics,* 14 May 2008, vol. 24 (13), 1549-1551
- **RAMAKERS et al.** *Neuroscience Letters,* 13 March 2003, vol. 339 (1), 62-66